# EUROPÄISCHE PATENTANMELDUNG

(11) **EP 4 837 596 A1**
(43) Veröffentlichungstag der Anmeldung: **11.11.2026**
(21) Anmeldenummer: 25175084.0
(22) Anmeldetag: 08.05.2025
(51) Int. Cl.: A61L 27/34, A61L 27/50

(54) **SCHICHTAUFBAU UND ANORDNUNG ZUR MINIMIERUNG VON REIBUNG UND ABRIEB IN WÄSSRIGEN MEDIEN**

(71) Anmelder: Institut für Bioprozess- und Analysenmesstechnik e.V., 37308 Heilbad Heiligenstadt (DE)
(72) Erfinder: LIEFEITH, Klaus, 37318 Uder (DE); ROTHE, Holger, 37327 Leinefelde-Worbis (DE); HILDEBRAND, Gerhard, 37318 Lutter (DE); BÖRKE, Julia Marie, 37115 Duderstadt (DE)
(74) Vertreter: Maikowski & Ninnemann Patentanwälte Partnerschaft mbB

(57) **Zusammenfassung**

Die Erfindung betrifft einen Schichtaufbau aufweisend
eine Substratschicht;
eine erste Schicht die kovalent an die Substratschicht gebunden ist aufweisend einen Linker;
eine zweite Schicht aufweisend einen vernetzten Polyelektrolyt-Multilayer, die an die erste Schicht gebunden ist;
wobei die zweite Schicht Polyzwitterionen oder hydrophile Polymere aufweist, die ein- oder angelagert sind. Weiterhin betrifft die Erfindung eine Anordnung die zwei erfindungsgemäße Schichtaufbauten aufweist, eine Gelenkkomponente mit dem erfindungsgemäßen Schichtaufbau und ein Verfahren zur Herstellung des erfindungsgemäßen Schichtaufbaus.

## Beschreibung

Die Erfindung betrifft einen Schichtaufbau aufweisend
eine Substratschicht;
eine erste Schicht die kovalent an die Substratschicht gebunden ist aufweisend einen Linker;
eine zweite Schicht aufweisend einen vernetzten Polyelektrolyt-Multilayer, die an die
erste Schicht gebunden ist;
wobei die zweite Schicht Polyzwitterionen oder hydrophile Polymere aufweist, die ein- oder angelagert sind. Weiterhin betrifft die Erfindung eine Anordnung die zwei erfindungsgemäße Schichtaufbauten aufweist, eine Gelenkkomponente mit dem erfindungsgemäßen Schichtaufbau und ein Verfahren zur Herstellung des erfindungsgemäßen Schichtaufbaus.

Der menschliche Körper enthält über 300 Gelenke, die unterschiedlichen dynamischen Belastungen ausgesetzt sind und idealerweise ein Leben lang eine hervorragende Gleitbewegung gewährleisten. Ein Gelenk besteht primär aus zwei Knochenbereichen, die mit hyalinem Knorpel überzogen sind. Diese osteochondralen Zonen werden von der Synovia (Gelenkflüssigkeit) umspült und mit Nährstoffen versorgt. Die Bewegung des Gelenkes wird durch das Zusammenspiel aus hyalinem Knorpel und Synovia nahezu reibungsfrei gehalten. Der Reibungskoeffizient liegt bei µ~0,001-0,03 und dies unter Belastungen, die 10-20 MPa erreichen können [1-4].

Bislang sind alle Versuche, diese Reibungskoeffizienten in technischen Systemen zu realisieren, fehlgeschlagen [5-8]. Andererseits sind die prinzipiellen tribologischen Wirkmechanismen im natürlichen Knorpel inzwischen gut verstanden, d.h., die grundlegenden Ansätze für eine Überführung der tribologischen Performance des natürlichen intakten Knorpels in die Technik stehen zur Verfügung. Es besteht darüber hinaus Konsens, dass die sehr guten tribologischen Eigenschaften durch den Effekt der 'Hydration-Lubrication' erreicht werden [9-12]. Ursächlich hierfür ist eine auf der Oberfläche der Reibpartner befindliche Zone hydratisierter Makromoleküle. Die eigentliche Gleitpaarung besteht daher nur untergeordnet aus Biomolekülen, sondern ist vielmehr als Reibungssystem zwischen zwei immobilisierten Wasserphasen zu verstehen. Aus diesem Fakt erklärt sich die herausragende Performance des Gelenkknorpels. Einerseits wird durch die stabile Hydratisierung eine hohe Drucktoleranz erzeugt, andererseits zeigen die Wassermoleküle der hydratisierten Biomoleküle ein flüssigkeitsähnliches Verhalten, womit sich die geringen Reibungskoeffizienten erklären lassen [13]. Als ursächlich für diese stabile Hydratisierung wurden bereits 1984 Phospholipide identifiziert [14]. Im Folgenden wurde erkannt, dass die zwitterionischen Kopfgruppen der Phospholipide, die Phosphorylcholine (PC) außerordentlich stabile Hydrathüllen aus bis zu 15 Moleküllagen bilden [14-19]. Im Weiteren wurden unterschiedliche molekulare Systeme und Kombinationen untersucht, mit Hilfe derer sich die Performance des hyalinen Knorpels erreichen lässt. Dioleoylphosphatidylcholin Doppelschichten konnten einen Reibungskoeffizienten von µ~0,02 jedoch nur bis zu einem Druck von bis zu 0,3 MPa erreichen [20]. Wang, Liu [21] erreichten unter höheren Druck von über 60 MPa einen Reibungskoeffizient von µ~0,02 in Kombination mit Hyaluronsäure (HA). Die aktuell beste Performance mit Reibungskoeffizienten von µ~0,001 bei maximal 10 MPa zeigen liposomale Kombinationen aus PC-Lipiden und HA [22]. Aufgrund dieser Erkenntnisse wurde das Modell der 'Hydration Lubrication' weiterentwickelt, eine Überführung in technisch nutzbare Beschichtungen existiert jedoch bislang nicht [2, 22]. Das derzeitige funktionelle Modell des hyalinen Knorpels geht von einem zonalen Aufbau aus, wobei die untere kollagenreiche Zone die stabile Verankerung zum Knochen sowie die Funktion einer hochelastischen Bettung übernimmt. Auf dieser Zone aufbauend erfolgt eine graduelle Substitution des Kollagens durch Lubricin, das Hyaluronsäure binden kann. Hyaluronsäure wiederum kann die für die Gleiteigenschaften verantwortlichen Phospholipide immobilisieren [5, 23-26]. Diese bilden eine viskose Zone, bestehend aus Mono- und Doppellagen [27-29]. Weiterhin wurden Phospholipid-Vesikel nachgewiesen, denen aufgrund der besseren Beweglichkeit im Gelenkspalt eine weitere Verringerung der Reibung zugeschrieben wird.

Nach dem derzeitigen Stand der implantologischen Technik bestehen Großgelenksimplantate in aller Regel aus einem Metall-Schaft, einem verbundenen Keramik- oder Metallkopf und einem Gegenlager aus hochmolekularem Polyethylen (HMPE). Von diesen Komponenten werden im Laufe der Jahre Mikropartikel abgerieben, die zu Entzündungen und zum nekrotischem Knochenabbau führen können [30, 31]. Dieser Abrieb ist einer der größten Risikofaktoren für das aseptische Versagen eines künstlichen Gelenkes. Diese Degradationsprozesse, die in aller Regel das verwendete HMPE betreffen, können zwar entweder durch die Verwendung von Oxidkeramik oder durch weitere Nachvernetzung des HMPE zu UHMPE um 42-93% (je nach Studie) verringert werden, allerdings weisen auch diese Gleitpaarungen noch erhebliche Mengen an Abrieb auf, so dass das Problem nicht als gelöst eingestuft werden kann [32-35].

In Japan wurde durch die Aktivitäten der Gruppe um Kazuhiko Ishihara Beschichtungen auf der Basis von zwitterionischen Polymeren entwickelt [36]. Das Ziel war es, den Abrieb und den reibungsbedingten Verschleiß im künstlichen Gelenk zu reduzieren, in dem wasseraffine Phosphorylcholine eingesetzt wurden. Im Ergebnis wurden Polymer-Brushes aus 2-Methacryloyloxyethyl-phosphorylcholine (MPC) auf vernetztem HMPE immobilisiert. Entsprechende Beschichtungen führten zu einer besseren Benetzbarkeit und zeigten optimierte Reibungskoeffizienten im Vergleich zu anderen getesteten Modifikationen [36]. Die Beschichtung reduzierte im klinischen Einsatz periimplantäre Osteolysen zwar signifikant [37], jedoch verwendet Ishihara ausschließlich das Polymer MPC, welches auf die Oberfläche aufgebracht wird. Die Eigenschaften von hyalinem Knorpel können daher nur sehr eingeschränkt nachgeahmt werden.

Ein anderer, bioinspirierter Ansatz verwendet bakterielle Nanocellulose (BNC), um das fibrilläre Kollagen im hyalinen Knorpel zu imitieren [8]. Der Ansatz begründet sich darin, dass sich Cellulose im Körper weitgehend bioinert verhält und nicht enzymatisch abgebaut werden kann [38, 39]. Um eine elastische Bettung zu gewährleisten, wurden synthetische Polymere (Polyvinylalkohol) eingebacht und die notwendige Wasseraffinität wurde durch Poly-2-acrylamido-2-methyl-1-propansulfonsäure "nachgerüstet". Dieses Gel zeigte äquivalente mechanische und tribologische Eigenschaften wie porciner Knorpel, wobei anzumerken ist, dass die Reibkoeffizienten von porcinem Knorpel zwei Größenordnungen höher liegen als die für humanen Knorpel. Die Herstellung ist jedoch äußerst umständlich. BNC muss erst in einem biotechnologischen Prozess hergestellt werden, wobei die schlechte Mechanik der BNC über synthetische Polymere "verbessert" werden muss. Diese erfordert zahlreiche einzelne Prozessschritte, die bei einer Anwendung das Produkt sehr teuer und damit unwirtschaftlich machen.

WO2007091521 betrifft ein Lagermaterial mit einer hohen Verschleißbeständigkeit zur Verwendung in einer feuchten Umgebung. Eine Polymerschicht wird auf eine Substratschicht aus einem Polymermaterial aufgepfropft, wobei die Polymerschicht Phosphorylcholin-Gruppen aufweist. Das Lagermaterial kann genutzt werde, um die Reibung in einem künstlichen Gelenk zu minimieren. Hierfür wird das Lagermaterial auf eine erste Komponente des künstlichen Gelenks aufgebracht, wodurch die Reibung zu einer zweiten korrespondierenden Komponente, hergestellt aus Keramik oder Metall reduziert wird. Diese Lösung weist jedoch die gleichen Nachteile auf, wie für Ishihara bereits beschrieben und ist daher wenig variabel und nur eingeschränkt für spezielle Anwendungen optimierbar.

EP 3 154 550 B1 betrifft ein Verfahren zum Reduzieren eines Reibungskoeffizienten einer Oberfläche. Das Verfahren umfasst das Binden mindestens eines wasserlöslichen Polymers auf der Oberfläche und das Inkontaktbringen des mindesten einen wasserlöslichen Polymers mit Liposomen. Bei diesem Verfahren ist über einen längeren Zeitraum eine schlechte mechanische Beständigkeit gegenüber Normal- und Scherkräften zu erwarten.

EP 2 380 600 B1 betrifft ein Polymergleitmaterial, bei dem auf ein Polymersubstrat eine Überzugsschicht aufgebracht wird, wobei die Überzugsschicht durch das Aufbringen und Vernetzen von Monomeren aufgebracht wird. Weiterhin wird eine künstliche Gelenkkomponente beschrieben, die das Polymergleitmaterial als Gleitschicht aufweist. Diese Lösung weist jedoch die gleichen Nachteile auf, wie für Ishihara bereits beschrieben und ist daher wenig variabel und nur eingeschränkt für spezielle Anwendungen optimierbar.

Die Minimierung von Reibung und Abrieb zwischen zwei Komponenten ist zudem nicht nur im Bereich der Endoprothetik von Interesse, sondern prinzipiell bei allen technischen Reibpaaren in einer wasserbasierten Umgebung.

Ausgehend vom Stand der Technik ist es die Aufgabe der vorliegenden Erfindung die Nachteile aus dem Stand der Technik zu beheben und ein Gleitmaterial zur Verfügung zu stellen, mit dem die Reibung und der Abrieb zwischen zwei Komponenten minimiert werden kann.

Diese Aufgabe löst die vorliegende Erfindung mit einem Schichtaufbau gemäß Anspruch 1, einer Anordnung gemäß Anspruch 11, einer künstlichen Gelenkkomponente gemäß Anspruch 13 und einem Verfahren gemäß Anspruch 14.

### Detaillierte Beschreibung

Erfindungsgemäß wird ein Schichtaufbau aufweisend
eine Substratschicht;
eine erste Schicht die kovalent an die Substratschicht gebunden ist aufweisend einen Linker; und
eine zweite Schicht aufweisend einen vernetzten Polyelektrolyt-Multilayer, die an die erste Schicht gebunden ist;
wobei die zweite Schicht Polyzwitterionen und/oder hydrophile Polymere aufweist, die ein- oder angelagert sind;
zur Verfügung gestellt.

Geeignete Linker sind dem Fachmann bekannt. In einer Ausführungsform der vorliegenden Erfindung ist der Linker ausgewählt aus der Gruppe aufweisend Silane, und Tetraetherlipide.

In einer Ausführungsform sind geeignete Silane ausgewählt aus der Gruppe der Trichlorsilane, Ethoxy-Silane und Methoxy-Silane, die jeweils über Aminogruppen, Carboxylgruppen, Acrylatgruppen, Methacrylatgruppen, oder Cyanatgruppen zur Anbindung der Schichtkomponenten der zweiten Schicht verfügen. In einer Ausführungsform ist das Silan ein 3-Aminopropyltriethoxysilan (APTES).

In einer Ausführungsform sind Tetraetherlipide ausgewählt aus der Gruppe aufweisend Caldarchaeol und dessen Derivate sowie Glycerol Dialkyl Nonitol Tetraether (GDNT) und dessen Derivate.

Erfindungsgemäß ist der Linker kovalent an die Substratschicht gebunden. Erfindungsgemäß ist der Linker an mindestens einen Abschnitt der Oberfläche der Substratschicht gebunden, muss diese aber nicht vollständig bedecken.

Erfindungsgemäß weist die Substratschicht eine Oberfläche auf, die Hydroxylgruppen zur Anbindung der Linker aufweist. An eine solche oxidische Oberfläche kann der Linker kovalent gebunden werden. In einer Ausführungsform der vorliegenden Erfindung weist die Substratschicht ein Material aus der Gruppe aufweisend Glas, Keramik, Metall, Metalllegierungen und Polymer auf oder besteht aus diesem. In einer Ausführungsform weist die Substratschicht ein Polymer auf oder besteht aus diesem und die Substratschicht ist mit einem Sauerstoffplasma oder einer Ozonierung behandelt, um Hydroxylgruppen zur Anbindung der Linker zu erzeugen.

Erfindungsgemäß weist der Schichtaufbau eine zweite Schicht auf, die auf der ersten Schicht angeordnet und an diese angebunden ist. Die zweite Schicht weist einen vernetzten Polyelektrolyt-Multilayer (PEM) auf oder besteht aus diesem.

Polyelektrolyt-Multilayer (PEM) entstehen durch das alternierende Aufbringen von entgegengesetzt geladenen Polymeren auf eine geladene Oberfläche. Dieses hat wiederum eine entgegengesetzte Ladung zu dem Polymer, das in der ersten Schicht aufgebracht wird. Eine Doppellage eines Polyelektrolyt-Multilayer besteht demnach aus einer anionischen Lage und einer kationischen Lage. In der vorliegenden Erfindung wird der Polyelektrolyt-Multilayer auf den Linker aufgebracht.

Der Polyelektrolyt-Multilayer ist aus Poly-Kationen und Poly-Anionen aufgebaut. In einer Ausführungsform sind die Poly-Kationen ausgewählt aus der Gruppe aufweisend
- Poly-Allylamin Hydrochlorid
- Poly-Anilin
- Poly-L-Lysin
- Dextran (aminiert)
- Poly-Allylamin
- Poly-ethylenimin.

In einer Ausführungsform sind die Poly-Anionen ausgewählt aus der Gruppe aufweisend
- Poly-Methacrylsäure
- Poly-Acrylsäure
- Poly-Styrene-4-Sulfonat
- Poly-Thiophen-3-Acetylsäure
- Poly(2-acrylamid-2-methyl-1-propansulfonsäure)
- Polyglutaminsäure
- Dextran (sulfatisiert)
- Heparin
- Hyaluronsäure.

Eine Doppellage des Polyelektrolyt-Multilayer weist in einer Ausführungsform jeweils genau eine Art eines Poly-Kations in der kationischen Lage und eine Art eines Poly-Anions in der anionischen Lage auf. In einer weitere Ausführungsform weist eine Doppellage eines Polyelektrolyt-Multilayers eine Mischung von Poly-Anionen in der anionischen Lage un/oder eine Mischung von Poly-Kationen in der kationischen Lage auf.

In einer Ausführungsform der vorliegenden Erfindung kann der Polyelektrolyt-Multilayer mehrere Doppellagen aufweisen, die aus verschiedenen Poly-Kationen und Poly-Anionen aufgebaut sind.

In einer bevorzugten Ausführungsform weist der Polyelektrolyt-Multilayer Poly-L-Lysin (PLL) als Poly-Kation und Polyglutaminsäure (PGA) als Poly-Anion auf.

Bevorzugt ist die zweite Schicht an der ersten Schicht kovalent angebunden. Hierfür wird eine erste Schicht der Polyelektrolyte kovalent an den Linker angebunden. Dies kann beispielsweise durch eine EDC/NHS vermittelte Peptidbindung oder photochemisch über Acrylat- und Methacrylatgruppen erfolgen. Auf die erste Schicht der Polyelektrolyte werden anschließend weitere Polyelektrolytschichten aufgebracht, wodurch der Polyelektrolyt-Multilayer gebildet wird.

In einer Ausführungsform weist der Polyelektrolyt-Multilayer zwischen 5 und 30 Doppellagen, bevorzugt zwischen 10 und 20 Doppellagen, besonders bevorzugt zwischen 13 und 17 Doppellagen auf.

Bevorzugt ist der Vernetzungsgrad der Polyelektrolyte der zweiten Schicht innerhalb der zweiten Schicht nicht überall gleich. Die zweite Schicht weist dabei eine erste Oberfläche auf, die mit der ersten Schicht verbunden ist und eine zweite Oberfläche, die der ersten Oberfläche gegenüber angeordnet ist. Die Vernetzung der Polyelektrolyte ist im Bereich der ersten Oberfläche am größten und nimmt innerhalb der zweiten Schicht zur zweiten Oberfläche hin ab. Prinzipiell ist ein Polyelektrolyt-Multilayer von Natur aus am Substrat am stärksten vernetzt und damit am kompaktesten und mit zunehmendem Abstand vom Substrat schwächer vernetzt und damit weicher. Dies ist eine intrinsische Eigenschaft des Polyelektrolyt-Multilayer. Bevorzugt ist der Polyelektrolyt-Multilayer vernetzt, um die mechanische Stabilität zu erhöhen.

In einer Ausführungsform kann der Polyelektrolyt-Multilayer nach dem Aufbringen auf die erste Schicht des Schichtaufbaus nachvernetzt werden, um die gewünschten Vernetzungsgrade in der zweiten Schicht zu erhalten. Eine Nachvernetzung kann wie folgt durchgeführt werden.

In einer Ausführungsform erfolgt die Nachvernetzung durch die Verwendung von Polyelektrolyten, die selber quervernetzt werden können. Die verwendeten Polyelektrolyte weisen dabei funktionelle Gruppen auf, die über dem Fachmann bekannte Methoden vernetzt werden können. Funktionelle Gruppen sind beispielsweise Carboxylgruppen, primäre Amine, Epoxidgruppen, Acrylatgruppen, Methacrylatgruppen, Hydroxylgruppen. Die für die Vernetzung notwendigen Agenzien werden während des Schichtauftrags der Beschichtungslösung beigemengt oder nachträglich in die Schicht über Diffusion eingebracht.

Mögliche Vernetzungsreaktionen sind beispielsweise Aldehyd-basierte Vernetzung mittels Glutaraldehyd oder Formaldehyd, Epoxid-basierte Vernetzung (z.B. Genipin), Carbodiimid basierte Vernetzung (EDC/NHS), Maleimid basierte Vernetzung sowie photochemische Vernetzung unter Verwendung von Photoinitiatoren.

In einer weiteren Ausführungsform erfolgt die Nachvernetzung durch kurzkettige Quervernetzer, die während des Schichtauftrags der Beschichtungslösung beigemengt oder nachträglich in die Schicht über Diffusion eingebracht werden. Geeignete kurzkettige Quervernetzer sind dem Fachmann bekannt und stammen aus den Gruppen der Di- oder Tri-acrylate bzw. Di- oder Tri-methacrylate. Beispielsweise sind Di- oder Tri-acrylate bis zu einem Molekulargewicht von ca. 5000 geeignet.

Für jede Form der photochemischen Nachvernetzung ist das gleichzeitige Einbringen eines Photoinitiators notwendig. Wird eine photochemische Nachvernetzung verwendet, weist der Polyelektrolyt-Multilayer daher weiterhin mindestens einen Photoinitiator, bevorzugt einen radikalischen Photoinitiator auf. Geeignete Photoinitiatoren sind dem Fachmann bekannt und können beispielsweise sein:
- Lithiumphenyl-2,4,6-trimethylbenzoylphosphinat (LAP)
- Irgacure 2959
- Rose Bengal
- Campherchinon
- Eosin Y
- Riboflavin
- Thioxanthone-Sulfonat
- Curcumin

Diese Aufzählung gibt lediglich Beispiele an. Dem Fachmann sind noch wesentlich mehr geeignete Photoinitiatoren bekannt.

Der Polyelektrolyt-Multilayer kann mittels eines Dipping Prozesses (Tauchverfahren) oder Spray Prozesses (Sprühprozess) aufgebaut werden. Hierfür wird eine Beschichtungslösung für den Tauch- oder Sprühprozess verwendet. Die Nachvernetzung kann dann erfindungsgemäß weiterhin wie folgt beeinflusst werden:
In einer Ausführungsform weist die Beschichtungslösung ein Vernetzungsreagenz auf. Geeignete Vernetzungsreagenzien, beispielsweise EDC (1-Ethyl-3-(3-dimethylaminopropyl)carbodiimin) sind dem Fachmann bekannt. Über die Konzentration in der Lösung für die jeweilige Lage kann die Konzentration in der Schicht und somit die Effizienz der Vernetzung gesteuert werden. Die Konzentration der Vernetzungsreagenz in der Beschichtungslösung liegt im Bereich von 1-100 mM, bevorzugt zwischen 25-75 mM.

In einer weiteren Ausführungsform wird die fertige Beschichtung nach dem Schichtaufbau mit einem Vernetzungsreagenz versetzt, um diese eindiffundieren zu lassen. Geeignete Vernetzungsreagenzien, beispielsweise EDC (1-Ethyl-3-(3-dimethylaminopropyl)carbodiimin) sind dem Fachmann bekannt. Die Konzentration der Vernetzungsreagenz in der Beschichtungslösung liegt im Bereich von 1-100 mM, bevorzugt zwischen 25-75 mM. Die Schichten werden dabei homogen mit dem Vernetzer infiltriert und homogen nachvernetzt.

In einer weiteren Ausführungsform weist die Beschichtungslösung einen kurzkettigen, photosensitiven Quervernetzer sowie einen Photoinitiator auf. Geeignete kurzkettige Quervernetzter wurden weiter oben bereits genannt. Auch hier kann über die Konzentration in der Beschichtungslösung die Konzentration und Effizienz der Vernetzung im Polyelektrolyt-Multilayer gesteuert werden. Die Konzentration des Quervernetzers in der Lösung liegt dabei im Bereich von 0,1-100 mM, bevorzugt zwischen 5-50 mM, besonders bevorzugt zwischen 10-20 mM. Die Konzentration des Photoinitiators in der Lösung liegt in einem Bereich von 0,01-10 Masseprozent, bevorzugt 0,1-1 Masseprozent.

In einer weiteren Ausführungsform wird die fertige Beschichtung nach dem Schichtaufbau mit einem kurzkettigen Quervernetzer sowie einem Photoinitiator versetzt, um diese eindiffundieren zu lassen. Die Konzentration des Quervernetzers in der Lösung liegt dabei im Bereich von 0,1-100 mM, bevorzugt zwischen 5-50 mM, besonders bevorzugt zwischen 10-20 mM. Die Konzentration des Photoinitiators in der Lösung liegt in einem Bereich von 0,01-10 Masseprozent, bevorzugt 0,1-1 Masseprozent. Die Schichten werden dabei homogen mit dem Vernetzer infiltriert und homogen nachvernetzt.

In einer weiteren Ausführungsform weist die Benetzungslösung ein Gemisch aus einem photovernetzbaren Poly-Kation, einem Photoinitiator und einem nicht vernetzbaren Poly-Kation für die Erzeugung der kationischen Lagen auf. Beim Schichtaufbau wird die Konzentration des vernetzbaren Poly-Kations variiert, wodurch die Vernetzung in der Schicht gesteuert werden kann. Die Konzentration des vernetzbaren Poly-Kations liegt in einem Bereich von 10-100% bezogen auf die Gesamtmenge von Poly-Kation. Bevorzugt liegt die Konzentration bei der ersten Lage Poly-Kation bei 100-50% und wird mit jeder weiteren Doppellage reduziert. Die obersten Lagen haben bevorzugt einen relativen Gehalt von 10% vernetzbarem Poly-Kation.

In einer weiteren Ausführungsform weist die Benetzungslösung ein Gemisch aus einem photovernetzbaren Poly-Anion, einem Photoinitiator und einem nicht vernetzbaren Poly-Anion für die Erzeugung der anionischen Lagen auf. Beim Schichtaufbau wird die Konzentration des vernetzbaren Poly-Anions variiert, wodurch die Vernetzung in der Schicht gesteuert werden kann. Die Konzentration des vernetzbaren Poly-Anions liegt in einem Bereich von 10-100% bezogen auf die Gesamtmenge von Poly-Anion. Bevorzugt liegt die Konzentration bei der ersten Lage Poly-Anion bei 100-50% und wird mit jeder weiteren Doppellage reduziert. Die obersten Lagen haben bevorzugt einen relativen Gehalt von 10% vernetzbarem Poly-Anion.

Der Vernetzungsgrad ist damit in der Nähe der Substratschicht am größten und liefert funktionell gesehen eine elastische Bettung. Zur zweiten Oberfläche der zweiten Schicht hin verringert sich der Vernetzungsgrad. Dadurch geht das vermehrt elastische Verhalten der hochvernetzten Bereiche in ein vermehrt viskoses Verhalten über. Mit dem Absenken des Vernetzungsgrades steigt ebenso die Quellbarkeit der Polyelektrolyte, sodass die Hydratisierung ansteigt, wenn der Schichtaufbau mit einem wässrigen Medium in Kontakt kommt.

Erfindungsgemäß weist die zweite Schicht hydrophile Polymere und/oder Polyzwitterionen auf, die bevorzugt durch Coulomb'sche Wechselwirkungen in die zweite Schicht eingelagert oder an diese angelagert sind. Dieser Effekt kann über die Konzentration der hydrophile Polymere und/oder Polyzwitterionen in der Benetzungslösung gesteuert werden. Die Konzentrationen der hydrophile Polymere und/oder Polyzwitterionen liegen in einer Ausführungsform zwischen 0,1 mg/ml und 10 mg/ml, bevorzugt zwischen 0,5 mg/ml und 5 mg/ml, besonders bevorzugt beträgt die Konzentration 1 mg/ml.

In einer Ausführungsform sind die Polyzwitterionen ausgewählt aus einer Gruppe aufweisend Phosphobetaine, Sulfobetaine, Carbobetaine.

In einer Ausführungsform sind die hydrophilen Polymere ausgewählt aus der Gruppe aufweisend Biopolymere wie Hyaluronsäure und synthetische Polymere wie Polyethylenglycol und Poly-Oxazoline.

Die erfindungsgemäße Zusammenführung von chemisch-kovalenten und Coulomb'schen Bindungen der Polyzwitterionen ist aus dem Stand der Technik nicht bekannt.

In einer Ausführungsform der Erfindung ist der Schichtaufbau Teil eines künstlichen Gelenkes oder einer künstlichen Gelenkkomponente. Künstliche Gelenke weisen im Allgemeinen zwei Gelenkkomponenten auf, die in Wechselwirkung miteinander die Funktion eines natürlichen Gelenks imitieren können. An der Stelle, an der die zwei Gelenkkomponenten aufeinandertreffen, entsteht eine sogenannte Gleitebene. Die Reibungskräfte, die zwischen den Gelenkkomponenten an dieser Stelle auftreten sorgen dafür, dass es zu einem unerwünschten Abrieb von Material an den Gelenkkomponenten kommen kann. Die Reibungskräfte sollten daher möglichst minimal sein.

Ist der Schichtaufbau Teil einer Gelenkkomponente, so bildet der Schichtaufbau eine Gleitschicht der Gelenkkomponente. Dies dient dazu, die Reibung und damit den Abrieb der einen Gelenkkomponente zu einer weiteren Gelenkkomponente zu minimieren.

In einer Ausführungsform ist der Reibungskoeffizient zwischen zwei Gelenkkomponenten unter 0,05, bevorzugt zwischen 0,01 und 0,001.

In einer Ausführungsform ist der erfindungsgemäße Schichtaufbau Teil eines künstlichen Gelenkes. In dieser Ausführungsform weist jede der Gelenkkomponenten einen erfindungsgemäßen Schichtaufbau auf, wobei die Schichtaufbauten jeweils zur Gleitebene hin angeordnet sind, so dass die Gleitebene zwischen dem Schichtaufbau der ersten Gelenkkomponente und dem Schichtaufbau der zweiten Gelenkkomponente gebildet wird. Die Reibungskräfte in der Gleitebene können so minimiert werden, womit auch der Materialabrieb der Substratschicht des ersten Schichtaufbaus und der Materialabrieb der Substratschicht des zweiten Schichtaufbaus verringert oder sogar unterbunden wird. In einer Ausführungsform ist der Reibungskoeffizient zwischen der Substratschicht des ersten Schichtaufbaus und der Substratschicht des zweiten Schichtaufbaus unter 0,05, bevorzugt zwischen 0,01 und 0,001.

In einer weiteren Ausführungsform ist der erfindungsgemäße Schichtaufbau Teil einer technischen Reibpaarung. In diesem Fall bilden zwei Bauteile über eine Gleitebene eine Reibpaarung, erfindungsgemäß weist mindestens eines der Bauteile einen erfindungsgemäßen Schichtaufbau auf, der an die Gleitebene angrenzt. In einer Ausführungsform weisen beide Bauteile der Reibpaarung einen erfindungsgemäßen Schichtaufbau auf, wobei die Schichtaufbauten beider Bauteile an die Gleitebene angrenzen und damit die Reibungskraft zwischen den Bauteilen minimiert werden kann.

Die Erfindung stellt eine künstliche Gelenkkomponente aufweisend einen erfindungsgemäßen Schichtaufbau zur Verfügung, wobei der Schichtaufbau die Gleitfläche der künstlichen Gelenkkomponente bildet.

Weiterhin umfasst die Erfindung eine Anordnung zur Minimierung von Reibung und Abrieb aufweisend einen ersten und einen zweiten erfindungsgemäßen Schichtaufbau, wobei sich der erste und zweite Schichtaufbau gegenüberliegen und eine Gleitebene einschließen und wobei die Gleitebene ein wässriges Gleitmedium aufweist.

In einer Ausführungsform ist das wässrige Gleitmedium ausgewählt aus der Gruppe aufweisend Synovia, artifizielle Synovia und Wasser.

Synovia ist eine viskose, klare Flüssigkeit, die Wasser, Hyaluronsäure, Protein, Lipide und Zelltrümmer enthält. Sie dient als natürliches Schmiermittel in Gelenken, Schleimbeuteln und Sehnenscheiden.

Synovia kann auch künstlich hergestellt werden. Verfahren hierfür sind dem Fachmann bekannt [40].

Durch die erfindungsgemäße Anordnung wird die Reibungskraft in der Gleitebene zwischen dem ersten Schichtaufbau und dem zweiten Schichtaufbau minimiert. Hierdurch kann der Materialabrieb der Substratschicht des ersten Schichtaufbaus und der Materialabrieb der Substratschicht des zweiten Schichtaufbaus minimiert oder gar unterbunden werden.

Weiterhin stellt die Erfindung ein Verfahren zur Herstellung eines erfindungsgemäßen Schichtaufbaus zur Verfügung, aufweisend die Schritte
- Bereitstellen einer Substratschicht;
- Kovalente Anbindung der Linkermoleküle der ersten Schicht des Schichtaufbaus;
- Anbinden der zweiten Schicht des erfindungsgemäßen Schichtaufbaus durch
   ∘ Anbinden einer ersten Polyelektrolytschicht an die Schicht aus Linkermolekülen durch EDC/NHS vermittelte Peptidbindung oder durch photochemische Anbindung; und
   ∘ Aufbau des Polyelektrolyt-Multilayers mittels eines Dipping Prozesses oder Spray Prozesses;
- Optional Nachvernetzung des Polyelektrolyt-Multilayers;
- Anlagerung und/oder Einlagerung von Polyzwitterionen und/oder hydrophilen Polymeren.

Alle Merkmale, die für die erfindungsgemäße Anordnung beschrieben wurden, treffen genauso auf das erfindungsgemäße Verfahren zu und umgekehrt.

In einer Ausführungsform werden in den Polyelektrolyt-Multilayer in einem weiteren Verfahrensschritt Poly-Anionen und/oder Poly-Kationen eingebracht.

Erfindungsgemäß wird zunächst eine Substratschicht zur Verfügung gestellt, an die eine Schicht des Linkers kovalent angebunden wird. Damit ist die erste Schicht des Schichtaufbaus auf der Substratschicht angeordnet. Dieser Verfahrensschritt kann beispielsweise durch einen Beschichtungsprozess erfolgen, bei dem der Linker (z.B. Silan) in einem Ethanol/Wasser Gemisch oder in Toluol gelöst wird und das Substrat für 12h eingelegt wird. Es bildet sich eine molekulare Monolage des Linkers (z.B. Silans) an der Oberfläche

Erfindungsgemäß wird in einem nächsten Schritt die zweite Schicht des Schichtaufbaus auf die erste Schicht aufgebracht. Hierfür wird eine erste Polyelektrolytschicht an die Schicht aus Linkermolekülen durch EDC/NHS vermittelte Peptidbindung oder photochemisch kovalent angebunden. EDC/NHS Crosslinking ist eine Standardmethode zur Vernetzung von Biomolekülen und dem Fachmann gut bekannt. Im nächsten Schritt wird der Polyelektrolyt-Multilayers aufgebaut. Bevorzugt wird dies mittels eines Dipping Prozesses oder einem Spray Prozess durchgeführt. Beim Dipping Prozess werden die einzelnen Schichten durch Eintauchen in entsprechende Polymerlösungen (Beschichtungslösung) aufgebracht. Beim Spray Prozess werden die einzelnen Schichten durch Aufsprühen der Polymerlösung (Beschichtungslösung) aufgebracht.

In einer Ausführungsform wird der Polyelektrolyt-Multilayer noch einmal nachvernetzt. Dies kann mittels der weiter oben beschriebenen Verfahren geschehen. Der Vernetzungsrad des Polyelektrolyt-Multilayers kann damit noch einmal verändert werden. Eine Nachvernetzung wird vor dem Anlagern und/oder Einbringen der Polyzwitterionen durchgeführt.

In einem weiteren Verfahrensschritt werden Polyzwitterionen in den Polyelektrolyt-Multilayer eingelagert oder an diesen angelagert. Dies kann durch einen weiteren Tauchschritt erfolgen. Die Anbindung erfolgt aufgrund der Coulomb'schen Wechselwirkungen.

Sämtliche Merkmale, die bereits für den erfindungsgemäßen Schichtaufbau, die erfindungsgemäße Anordnung und die künstliche Gelenkkomponenten beschrieben wurden, gelten im gleichen Maße für das erfindungsgemäße Verfahren und umgekehrt.

Die Erfindung stellt damit eine Lösung bereit, um die Gleiteigenschaften eines Reibungspaares aufrechtzuerhalten und gleichzeitig eine hinreichende mechanische Pufferung zur Verfügung zu stellen. Die mechanische Pufferung wird durch den Verlauf des Vernetzungsgrads innerhalb des Polyelektrolyt-Multilayers realisiert, während die Gleiteigenschaften durch die hohe Hydratisierung (aufgrund geringer Vernetzungsgrade) und die Polyzwitterionen an der zweiten Oberfläche der zweiten Schicht bereitgestellt werden.

In der Endoprothetik kann damit eine völlig neuartige Generation von Gelenkimplantaten zur Verfügung gestellt werden. Zudem kann die Erfindung genutzt werden, um im Bereich der technischen Reibpaarung schmiermittelfreie Reibpaarungen zur Verfügung zu stellen, was aus ökonomisch und ökologischer Sicht ein deutlicher Gewinn zum Stand der Technik ist.

Im Folgenden wird die Erfindung durch 8 Figuren und 5 Ausführungsbeispiele näher erläutert.
- Figur 1: stellt den makromolekularen Aufbau eines hyalinen Knorpels dar;
- Figur 2: stellt schematische den Aufbau eines erfindungsgemäßen Schichtaufbaus dar;
- Figur 3: (A) bis (C) stellen schematisch die kovalente Kopplung eines Linkers an eine Substratschicht und die Anbindung einer ersten Polyelektrolytschicht dar;
- Figur 4: (A) und (B) stellen experimentelle Ergebnisse von Benetzungsmessungen und Zetapotentialmessungen dar;
- Figur 5: (A) und (B) stellen eine Bestimmung der Schichtdicke einer Lage (eines Layers) des Polyelektrolyt-Multilayers dar;
- Figur 6: (A) stellt den Reibungskoeffizienten verschiedener Schichtaufbauten dar; (B) stellt die prozentuale Reduktion der Reibung bezogen auf Substratschichten ohne den erfindungsgemäße Schichtaufbau dar;
- Figur 7: stellt den Reibungskoeffizienten währen eines Belastungstests dar.

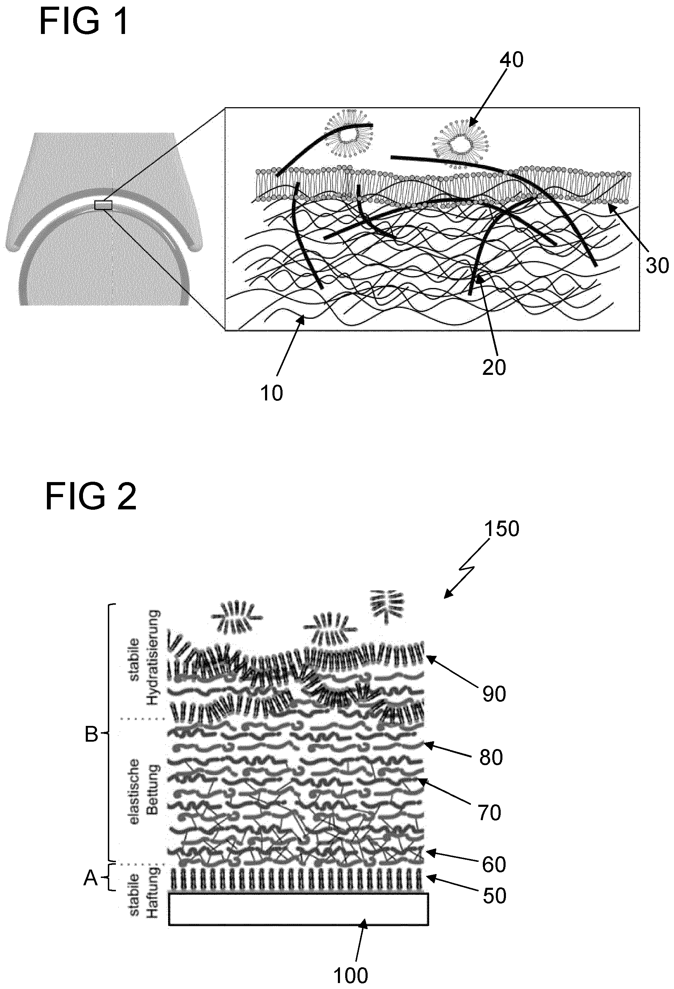

**Figur 1** stellt den makromolekularen Aufbau eines hyalinen Knorpels dar. Im vergrößerten Ausschnitt ist das Netzwerk aus Kollagen 10 zu erkennen in welchem Lubricin 20 angeordnet ist. Auf der Oberfläche befindet sich Hyaluronsäure 30 und Phopholipide 40. Das derzeitige funktionelle Modell des hyalinen Knorpels geht von einem zonalen Aufbau aus, wobei die untere kollagenreiche Zone 10 die stabile Verankerung zum Knochen sowie die Funktion einer hochelastischen Bettung übernimmt. Auf dieser Zone aufbauend erfolgt eine graduelle Substitution des Kollagens 10 durch Lubricin 20, das Hyaluronsäure 30 binden kann. Hyaluronsäure 30 wiederum kann die für die Gleiteigenschaften verantwortlichen Phospholipide 40 immobilisieren [5, 23-26]. Diese bilden eine viskose Zone, bestehend aus Mono- und Doppellagen [27-29]. Weiterhin wurden Phospholipid-Vesikel nachgewiesen, denen aufgrund der besseren Beweglichkeit im Gelenkspalt eine weitere Verringerung der Reibung zugeschrieben wird.

**Figur 2** stellt schematisch den Aufbau eines erfindungsgemäßen Schichtaufbaus 150 dar. Auf die Substratschicht 100 ist die erste Schicht A in Form eines Linkers 50 aufgebracht. Am Linker angeordnet befindet sich die zweite Schicht B, welche aus einem Polyelektrolyt-Multilayer besteht, der aus Monolagen von Poly-Anionen 80 und Monolagen von Poly-Kationen 70 aufgebaut ist. An der Oberfläche des Polyelektrolyt-Multilayer die von der Substratschicht abgewandt ist, sind Polyzwitterionen 90 an- oder eingelagert. Die zweite Schicht B weist eine Vernetzung 60 auf, die im Bereich der Substratschicht 100 am größten ist und zur Oberfläche, an der sich die Polyzwitterionen 90 befinden, hin abnimmt.

Durch diesen Aufbau des erfindungsgemäßen Schichtaufbaus 150 entsteht im Bereich der Substratschicht 100 eine elastische Bettung, während die Oberfläche der zweiten Schicht B mit den Polyzwitterionen 90 eine stabile Hydratisierung gewährleistet.

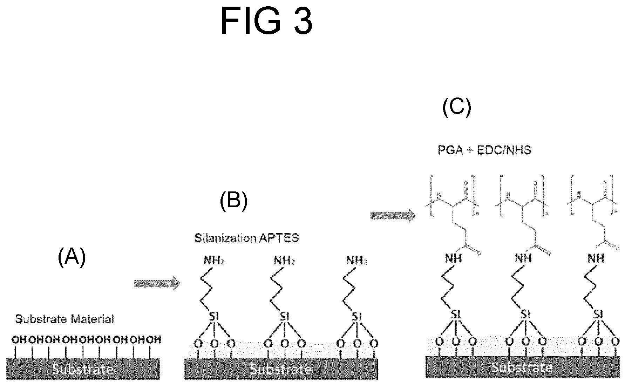

**Figuren 3 bis 7** werden im Rahmen der Ausführungsbeispiele näher erläutert.

### Ausführungsbeispiel 1

Das Ausführungsbeispiel betrifft die ersten Schritte der Herstellung eines erfindungsgemäßen Schichtaufbaus, wobei
- Borosilikatglas (Handelsname: Borofloat^{®} 33) als Substratschicht diente,
- 3-Aminopropyltriethoxysilan (APTES) als Linker der ersten Schicht;
- Polyglutaminsäure (PGA) als Polyelektrolyt der ersten Lage der zweiten Schicht.

Figur 3 (A) stellt schematisch die Substratschicht dar, an die eine Schicht Linker kovalent angebunden wurde (Figur3 (B)). An den Linker wurde eine Schicht Polyglutaminsäure über eine EDC/NHS vermittelte Peptidbindung kovalent angebunden (Figur 3(C)).

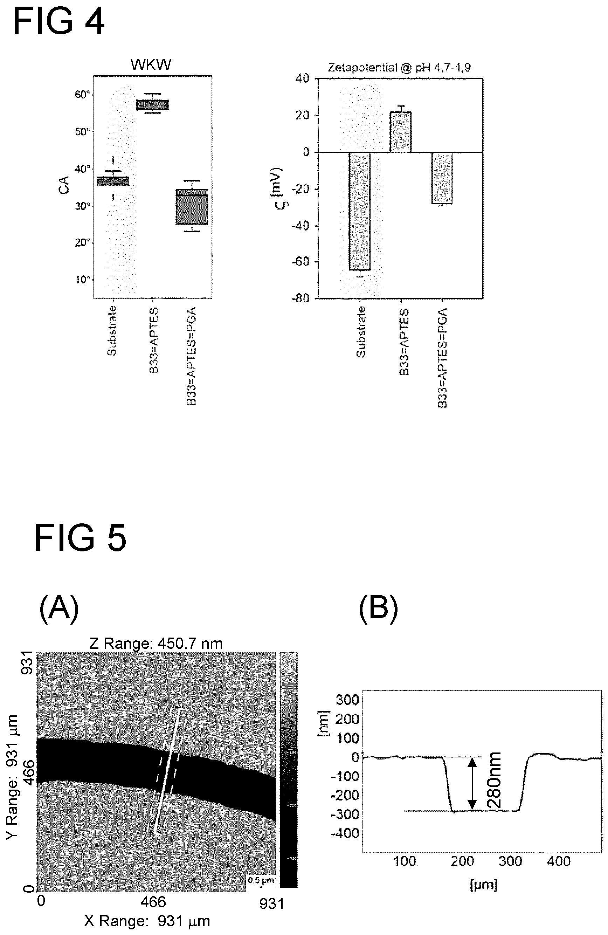

Der Nachweis der einzelnen Beschichtungsschritte erfolgte über die Messung des Wasserkontaktwinkels (WKW) und Zetapotenzial-Messungen (ZP) bei pH 4,8. Figur 4 (A) stellt den Verlauf des WKW und Figur 4 (B) ZP als Nachweis der Änderung der Benetzbarkeit sowie der Ladungsänderung an der Oberfläche durch die einzelnen Beschichtungsschritte dar. Die Messungen wurden jeweils für
a) die Substratschicht allein (entsprechend schematischem Aufbau gemäß Figur 3 (A)),
b) die Substratschicht mit angebundenem Linker (entsprechend schematischem Aufbau gemäß Figur 3 (B)); und
c) die Substratschicht mit angebundenem Linker und erster Schicht Polyglutaminsäure (entsprechend schematischem Aufbau gemäß Figur 3 (C))
durchgeführt.

Das oxidische Substrat besitzt bei pH 4,8 eine negative Überschussladung, welche durch die kovalente Anbindung von APTES kompensiert wird. APTES besitzt durch die Aminogruppen einen kationischen Charakter, das ZP wir positiv. Gleichzeitig steigt der KW auf ca. 60°, ein aus Erfahrungswerten charakteristischer Wert für die Beschichtung mit APTES. Durch die Anbindung von Polyglutaminsäure wird diese positive Überschussladung wiederum durch die Carboxylgruppen des PGA kompensiert.

Gleichzeitig fällt der Kontaktwinkel durch die Beschichtung mit dem polaren Makromolekül auf ca. 30°.

In dem Ausführungsbeispiel konnte gezeigt werden, dass eine Anbindung der einzelnen Schichten des Schichtaufbaus erfolgreich durchgeführt werden kann und experimentell überprüfbar ist.

### Ausführungsbeispiel 2

Ein Aufbau wie in Ausführungsbeispiel 1 beschrieben, wurde genutzt und an die erste Lage des Polyelektrolyten weitere Lagen angefügt, um den Polyelektrolyt-Multilayer (PEM) aufzubauen.

Der Aufbau des Polyelektrolyt-Multilayers (PEM) erfolgte mittels eines automatisierten Dipping Prozesses. Es wurden 15 Doppel-Lagen aus den jeweiligen Polykationen und Polyanionen aufgetragen. In diesem Ausführungsbeispiel wurde hierfür Polyglutaminsäure und Poly-L-Lysin genutzt. Im Ausführungsbeispiel erfolgte keine weitere zusätzliche Vernetzung des PEM.

Figur 5 (A) zeigt den Nachweis der erfolgreichen Beschichtung. Hierfür wurde der PEM mittels Nanotribometer (Pin-on-Disc) unter sehr hohen Lasten gezielt ablatiert. Die sich ergebende topografische Stufe wurde mittels Weißlichtinterferometrie am gequollenen PEM vermessen (Figur 5 (B)). Es konnte eine Schichtdicke von 280 nm für den Polyelektrolyt-Multilayer gemessen werden.

### Ausführungsbeispiel 3

Ein Aufbau wie in Ausführungsbeispiel 1 beschrieben wurde genutzt und an die erste Lage des Polyelektrolyten weitere Lagen angefügt, um den Polyelektrolyt-Multilayer (PEM) aufzubauen.

Der Aufbau des Polyelektrolyt-Multilayers (PEM) erfolgte mittels eines automatisierten Dipping Prozesses. Es wurden 15 Doppel-Lagen aus den jeweiligen Polykationen und Polyanionen aufgetragen. In diesem Ausführungsbeispiel wurde hierfür Polyglutaminsäure und Poly-L-Lysin genutzt.

Als Polyelektrolyt-Backbone wurde neben den genannten Polyelektrolyten (PGA und PLL) auch Poly-Allylamin-Hydrochlorid (PAH) als zusätzliches Poly-Kation sowie Hyaluronsäure [HA] als Poly-Anion eingebracht. Weiterhin wurde Phosphorylcholin (PC) als Polyzwitterionen in den PEM eingebracht, bzw. auf diesen aufgebracht. Die Beschichtungslösung wies eine Konzentration von 1 mg/ml für jede der genannten Komponenten auf.

### Ausführungsbeispiel 4

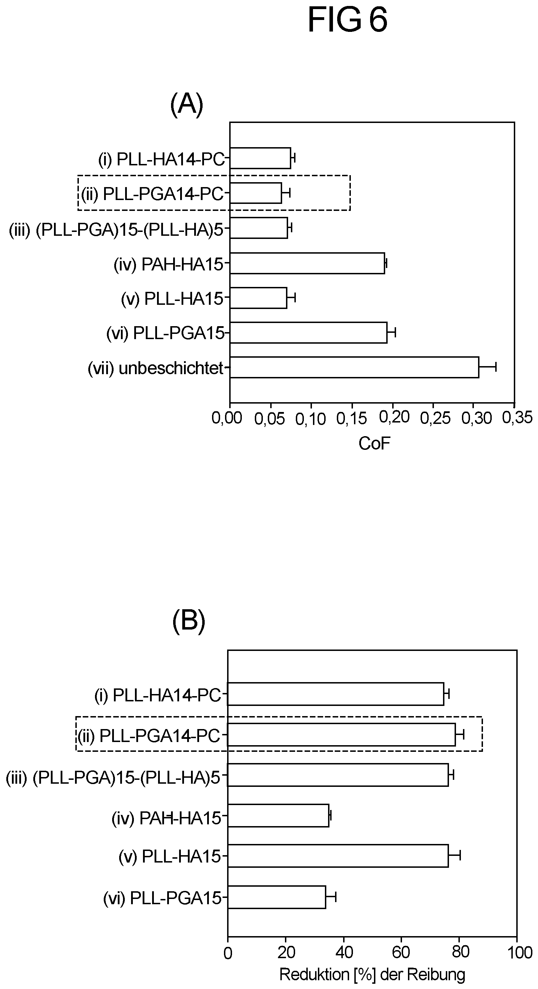

Es wurden folgende verschiedene Schichtaufbauten hergestellt:
Als Substratschicht diente jeweils Borosilikatglas und als Linker wurde APTES verwendet. Der Polyelektrolyte-Multilayer wies in den Schichtaufbauten (i) bis (vi) folgendes auf:
(i) 14 Doppellagen aus Poly-L-Lysin (PLL) als Poly-Kation und Hyaluronsäure (HA) als Poly-Anion sowie Phosphorylcholin (PC) als Polyzwitterionen (abgekürzt mit PLL-HA14-PC in Figur 6)
(ii) 14 Doppellagen aus Poly-L-Lysin (PLL) als Poly-Kation und Polyglutaminsäure (PGA) als Poly-Anion sowie Phosphorylcholin (PC) als Polyzwitterionen (abgekürzt mit PLL-PGA14-PC in Figur 6)
(iii) 15 Doppellagen aus Poly-L-Lysin (PLL) als Poly-Kation und Polyglutaminsäure (PGA) als Poly-Anion sowie 5 Doppellagen aus Poly-L-Lysin (PLL) als Poly-Kation und Hyaluronsäure (HA) als Poly-Anion, sowie als hydrophiles Polymer (abgekürzt mit ((PLL-PGA)15-(PLL-HA)5) in Figur 6)
(iv) 15 Doppellagen aus Poly-Allylamin-Hydrochlorid (PAH) als Poly-Kation und Hyaluronsäure (HA) als Poly-Anion, sowie als hydrophiles Polymer (abgekürzt mit PAH-HA15 in Figur 6)
(v) 15 Doppellagen aus Poly-L-Lysin (PLL) als Poly-Kation und Hyaluronsäure (HA) als Poly-Anion, sowie als hydrophiles Polymer (abgekürzt mit PLL-HA15 in Figur 6)
(vi) 15 Doppellagen aus Poly-L-Lysin (PLL) als Poly-Kation und Polyglutaminsäure (PGA) als Poly-Anion (abgekürzt mit PLL-PGA15 in Figur 6)

Die Schichtaufbauten (i) bis (v) stellen erfindungsgemäße Schichtaufbauten dar. Die mit (vii) gekennzeichnete Messung in Figur 6 (A) wurde an einer unbeschichteten Substratschicht durchgeführt.

Mittels Nanotribometer wurden in Pin-on-Disc Versuchen die Reibkoeffizienten bei einer mechanischen Normalkraft von 10 mN für alle Schichtaufbauten (i) bis (vi) sowie für eine unbeschichtet Substratschicht (vii) ermittelt. Die Reibpaarung bestand jeweils aus dem Schichtaufbau als planarer Reibpartner und einer unbeschichteten Kugel aus Zirkonoxid (Durchmesser: 2 mm). Als Schmiermedium wurde eine artifizielle Synovia in Anlehnung an Bortel et al. [40] verwendet.

Figur 6 (A) stellt die ermittelten Reibbeiwerte dar. Es ist zu erkennen, dass die erfindungsgemäßen Schichtaufbauten (ii) und (v) den geringsten Reibwert zeigt. Figur 6 (B) stellt die erreichte prozentuale Reibungsminimierung bezogen auf die unbeschichteten Reibpaarungen dar.

### Ausführungsbeispiel 5

Der Schichtaufbau gemäß (ii) des Ausführungsbeispiels 4 wurde einem Belastungstest unterzogen, um dessen Stabilität zu testen.

Die Stabilität konnte über die folgende Prozedur demonstriert werden:
1. Standard-Test-Prozedur (STP) zur Ermittlung des Reibkoeffizienten unter Variation der Geschwindigkeit von 40 mms⁻¹ bis 1 mms⁻¹ bei 10 MPa (insgesamt 275 Zyklen von je 18,85 mm)
2. 10.000 Zyklen (18,85 mm) bei 10 MPa und 40 mms-1
3. Waschen in Ethanol(abs.)
4. Waschen in Wasser (deion.)
5. Trocknen im Luftstrom
6. Lagerung für 7d trocken
7. Rückquellung in Wasser
8. 10.000 Zyklen (18,85 mm) bei 10 MPa und 40 mms⁻¹

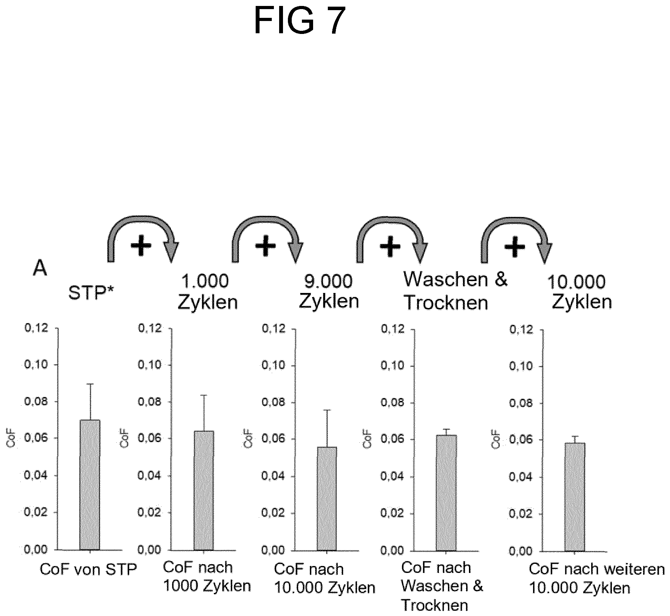

Es konnte demonstriert werden, dass das Schichtsystem auch nach dem o.g. Belastungstest keine Degradation und keinen nennenswerten Verlust der Reibungsminimierung aufweist (vgl. Figur 7).

### Referenzen

1. Forster, H. and J. Fisher, The influence of loading time and lubricant on the friction of articular cartilage. Proceedings of the Institution of Mechanical Engineers, Part H: Journal of Engineering in Medicine, 1996. 210(2): p. 109-119.
2. Jahn, S., J. Seror, and J. Klein, Lubrication of Articular Cartilage. Annu Rev Biomed Eng, 2016. 18: p. 235-58.
3. Hodge, W., et al., Contact pressures in the human hip joint measured in vivo. Proceedings of the National Academy of Sciences, 1986. 83(9): p. 2879-2883.
4. Afoke, N.Y., P.D. Byers, and W.C. Hutton, Contact pressures in the human hip joint. J Bone Joint Surg Br, 1987. 69(4): p. 536-41.
5. Schmidt, T.A., et al., Boundary lubrication of articular cartilage: role of synovial fluid constituents. Arthritis & Rheumatism, 2007. 56(3): p. 882-891.
6. Chang, D.P., et al., Friction force microscopy of lubricin and hyaluronic acid between hydrophobic and hydrophilic surfaces. Soft Matter, 2009. 5(18): p. 3438-3445.
7. Das, S., et al., Synergistic interactions between grafted hyaluronic acid and lubricin provide enhanced wear protection and lubrication. Biomacromolecules, 2013. 14(5): p. 1669-1677.
8. Yang, F., et al., A Synthetic Hydrogel Composite with the Mechanical Behavior and Durability of Cartilage. Advanced Functional Materials, 2020: p. 2003451.
9. Briscoe, W.H., et al., Boundary lubrication under water. Nature, 2006. 444(7116): p. 191-194.
10. Klein, J., Hydration lubrication. Friction, 2013. 1(1): p. 1-23.
11. Jin, Z. and D. Dowson, Elastohydrodynamic lubrication in biological systems. Proceedings of the Institution of Mechanical Engineers, Part J: Journal of Engineering Tribology, 2005. 219(5): p. 367-380.
12. Goldberg, R., et al., Boundary lubricants with exceptionally low friction coefficients based on 2D close-packed phosphatidylcholine liposomes. Advanced Materials, 2011. 23(31): p. 3517-3521.
13. Ma, L., et al., Origins of hydration lubrication. Nature communications, 2015. 6(1): p. 1-6.
14. Hills, B. and B. Butler, Surfactants identified in synovial fluid and their ability to act as boundary lubricants. Annals of the rheumatic diseases, 1984. 43(4): p. 641-648.
15. Sarma, A., G. Powell, and M. LaBerge, Phospholipid composition of articular cartilage boundary lubricant. Journal of orthopaedic research, 2001. 19(4): p. 671-676.
16. Daniel, D., et al., Hydration lubrication of polyzwitterionic brushes leads to nearly friction-and adhesion-free droplet motion. Communications Physics, 2019. 2(1): p. 1-7.
17. Yaseen, M., et al., The structure of zwitterionic phosphocholine surfactant monolayers. Langmuir, 2006. 22(13): p. 5825-5832.
18. Pabst, G., et al., Structural information from multilamellar liposomes at full hydration: full q-range fitting with high quality x-ray data. Physical Review E, 2000. 62(3): p. 4000.
19. Nagle, J.F., et al., X-ray structure determination of fully hydrated L alpha phase dipalmitoylphosphatidylcholine bilayers. Biophysical journal, 1996. 70(3): p. 1419-1431.
20. Trunfio-Sfarghiu, A.-M., et al., Role of nanomechanical properties in the tribological performance of phospholipid biomimetic surfaces. Langmuir, 2008. 24(16): p. 8765-8771.
21. Wang, M., et al., Hyaluronan and phospholipid association in biolubrication. Biomacromolecules, 2013. 14(12): p. 4198-4206.
22. Seror, J., et al., Supramolecular synergy in the boundary lubrication of synovial joints. Nature communications, 2015. 6(1): p. 1-7.
23. Minns, R. and F. Steven, The collagen fibril organization in human articular cartilage. Journal of anatomy, 1977. 123(Pt 2): p. 437.
24. Aspden, R. and D. Hukins, Collagen organization in articular cartilage, determined by X-ray diffraction, and its relationship to tissue function. Proceedings of the Royal Society of London. Series B. Biological Sciences, 1981. 212(1188): p. 299-304.
25. Jones, A.R., et al., Binding and localization of recombinant lubricin to articular cartilage surfaces. Journal of orthopaedic research, 2007. 25(3): p. 283-292.
26. Radin, E.L., D.A. Swann, and P.A. Weisser, Separation of a hyaluronate-free lubricating fraction from synovial fluid. Nature, 1970. 228(5269): p. 377-378.
27. Lin, W., et al., The Role of Hyaluronic Acid in Cartilage Boundary Lubrication. Cells, 2020. 9(7).
28. Zhu, L., et al., Ultra-low friction between boundary layers of hyaluronanphosphatidylcholine complexes. Acta biomaterialia, 2017. 59: p. 283-292.
29. Crescenzi, V., A. Taglienti, and I. Pasquali-Ronchetti, Supramolecular structures prevailing in aqueous hyaluronic acid and phospholipid vesicles mixtures: an electron microscopy and rheometric study. Colloids and Surfaces A: Physicochemical and Engineering Aspects, 2004. 245(1-3): p. 133-135.
30. Jacobs, J.J., et al., Osteolysis: basic science. Clinical Orthopaedics and Related Research®, 2001. 393: p. 71-77.
31. Glant, T.T., et al., Bone resorption activity of particulate-stimulated macrophages. Journal of Bone and Mineral Research, 1993. 8(9): p. 1071-1079.
32. Collier, J.P., et al., Comparison of cross-linked polyethylene materials for orthopaedic applications. Clinical Orthopaedics and Related Research (1976-2007), 2003. 414: p. 289-304.
33. Muratoglu, O.K., et al., A novel method of cross-linking ultra-high-molecular-weight polyethylene to improve wear, reduce oxidation, and retain mechanical properties: recipient of the 1999 HAP Paul Award. The Journal of arthroplasty, 2001. 16(2): p. 149-160.
34. Capello, W.N., et al., Alternative bearing surfaces: alumina ceramic bearings for total hip arthroplasty, in Bioceramics and Alternative Bearings in Joint Arthroplasty. 2005, Springer. p. 87-94.
35. Wroblewski, B., P. Siney, and P. Fleming, Low-friction arthroplasty of the hip using alumina ceramic and cross-linked polyethylene: a 17-year follow-up report. The Journal of bone and joint surgery. British volume, 2005. 87(9): p. 1220-1221.
36. Kyomoto, M., et al., Biomimetic hydration lubrication with various polyelectrolyte layers on cross-linked polyethylene orthopedic bearing materials. Biomaterials, 2012. 33(18): p. 4451-4459.
37. Takatori, Y., et al., Clinical and radiographic outcomes of total hip replacement with poly (2-methacryloyloxyethyl phosphorylcholine)-grafted highly cross-linked polyethylene liners: three-year results of a prospective consecutive series. Modern Rheumatology, 2015. 25(2): p. 286-291.
38. Farr, J., et al., High failure rate of a decellularized osteochondral allograft for the treatment of cartilage lesions. The American journal of sports medicine, 2016. 44(8): p. 2015-2022.
39. McLaughlin, S., et al., Injectable human recombinant collagen matrices limit adverse remodeling and improve cardiac function after myocardial infarction. Nature Communications, 2019. 10(1): p. 4866.
40. Bortel, E.L., B. Charbonnier, and R. Heuberger Development of a Synthetic Synovial Fluid for Tribological Testing. Lubricants, 2015. 3, 664-686 DOI: 10.3390/lubricants3040664.

### Bezugszeichenliste

- 10: Kollagen
- 20: Lubricin
- 30: Hyaluronsäure
- 40: Phospholipide
- 50: Linker
- 60: Vernetzung
- 61: hochvernetzter Bereich des Polyelektrolyt-Multilayers
- 62: gering vernetzter Bereich des Polyelektrolyt-Multilayers
- 70: Poly-Kation
- 80: Poly-Anion
- 90: Polyzwitterionen
- 100: Substratschicht
- 150: erfindungsgemäßer Schichtaufbau
- 200: erfindungsgemäße Anordnung
- A: erste Schicht
- B: zweite Schicht
- C: erfindungsgemäßer Schichtaufbau
- D: erfindungsgemäßer Schichtaufbau

## Patentansprüche

1. Schichtaufbau aufweisend
eine Substratschicht;
eine erste Schicht die kovalent an die Substratschicht gebunden ist aufweisend einen Linker;
eine zweite Schicht aufweisend einen vernetzten Polyelektrolyt-Multilayer die an die erste Schicht gebunden ist;
wobei die zweite Schicht Polyzwitterionen und/oder hydrophile Polymere aufweist, die ein- oder angelagert sind.

2. Schichtaufbau gemäß Anspruch 1 **dadurch gekennzeichnet, dass** der Linker ausgewählt ist aus der Gruppe aufweisend Silane, Tetraetherlipide.

3. Schichtaufbau gemäß einem der vorangegangenen Ansprüche, **dadurch gekennzeichnet, dass** der Polyelektrolyt-Multilayer Poly-Kationen und Poly-Anionen aufweist, wobei die Poly-Kationen ausgewählt sind aus der Gruppe aufweisend Poly-Allylamin Hydrochlorid (PAH), Poly-Anilin, Poly-L-Lysin (PLL), Dextran (aminiert), Poly-Allylamin, Poly-ethylenimin und die Poly-Anionen ausgewählt sind aus der Gruppe aufweisend Poly-Methacrylsäure, Poly-Acrylsäure, Poly-Styrene-4-Sulfonat, Poly-Thiophen-3-Acetylsäure, Poly(2-acrylamid-2-methyl-1-propansulfonsäure), Polyglutaminsäure (PGA), Dextran (sulfatisiert), Heparin und Hyaluronsäure (HA).

4. Schichtaufbau gemäß einem der vorangegangenen Ansprüche, **dadurch gekennzeichnet, dass** der Polyelektrolyt-Multilayer aus Poly-L-Lysin (PLL) als Poly-Kationen und Polyglutaminsäure (PGA) als Poly-Anionen besteht.

5. Schichtaufbau gemäß einem der vorangegangenen Ansprüche, **dadurch gekennzeichnet, dass** die zweite Schicht eine erste Oberfläche aufweist, die mit der ersten Schicht verbunden ist und eine zweite Oberfläche, die der ersten Oberfläche gegenüber angeordnet ist, wobei der Vernetzungsgrad der Polyelektrolyte im Bereich der ersten Oberfläche am größten ist und innerhalb der zweiten Schicht zur zweiten Oberfläche hin abnimmt.

6. Schichtaufbau gemäß einem der vorangegangenen Ansprüche, **dadurch gekennzeichnet, dass** der Polyelektrolyt-Multilayer zwischen 5 und 30 Doppellagen, bevorzugt zwischen 10 und 20 Doppellagen, besonders bevorzugt zwischen 13 und 17 Doppellagen aufweist.

7. Schichtaufbau gemäß einem der vorangegangenen Ansprüche, **dadurch gekennzeichnet, dass** die Polyzwitterionen ausgewählt sind aus einer Gruppe aufweisend Phosphobetaine, Sulfobetaine und Carbobetaine.

8. Schichtaufbau gemäß einem der vorangegangenen Ansprüche, **dadurch gekennzeichnet, dass** die hydrophilen Polymere ausgewählt sind aus der Gruppe aufweisend Biopolymere wie Hyaluronsäure und synthetische Polymere wie Polyethylenglycol und Poly-Oxazoline.

9. Schichtaufbau gemäß einem der vorangegangenen Ansprüche, **dadurch gekennzeichnet, dass** die Substratschicht ein Material aus der Gruppe aufweisend Glas, Keramik, Metall, Metalllegierung und Polymer aufweist oder aus diesem besteht.

10. Schichtaufbau gemäß einem der vorangegangenen Ansprüche, **dadurch gekennzeichnet, dass** der Schichtaufbau Teil eines künstlichen Gelenkes oder einer künstlichen Gelenkkomponente ist.

11. Anordnung zur Minimierung von Reibung und Abrieb aufweisend einen ersten und einen zweiten Schichtaufbau gemäß einem der Ansprüche 1 bis 10, wobei sich der erste und zweite Schichtaufbau gegenüberliegen und eine Gleitebene einschließen; und wobei die Gleitebene ein wässriges Gleitmedium aufweist.

12. Anordnung gemäß Anspruch 11, **dadurch gekennzeichnet, dass** das wässrige Gleitmedium ausgewählt ist aus der Gruppe aufweisend Synovia, artifizielle Synovia und Wasser.

13. Künstliche Gelenkkomponente aufweisend einen Schichtaufbau gemäß einem der Ansprüche 1 bis 10, wobei der Schichtaufbau die Gleitfläche der künstlichen Gelenkkomponente bildet.

14. Verfahren zur Herstellung eines Schichtaufbaus gemäß Anspruch 1 bis 10, aufweisend die Schritte
• Bereitstellen einer Substratschicht;
• Kovalente Anbindung der Linkermoleküle der ersten Schicht des Schichtaufbaus;
• Anbinden der zweiten Schicht des erfindungsgemäßen Schichtaufbaus durch
∘ Anbinden einer ersten Polyelektrolytschicht an die Schicht aus Linkermolekülen durch EDC/NHS vermittelte Peptidbindung oder durch photochemische Anbindung; und
∘ Aufbau des Polyelektrolyt-Multilayers mittels eines Dipping Prozesses oder eines Spray Prozesses;
• Optional Nachvernetzung des Polyelektrolyt-Multilayers;
• Anlagerung und/oder Einlagerung von Polyzwitterionen und/oder hydrophilen Polymeren.

15. Verfahren gemäß Anspruch 14, **dadurch gekennzeichnet, dass** weiterhin Poly-Anionen und/oder Poly-Kationen in den Polyelektrolyt-Multilayer eingebracht werden.
